Europäisches Patentamt

European Patent Office

Office européen des brevets

(19)

(11) Veröffentlichungsnummer: **0 105 863**
**B1**

(12) **EUROPÄISCHE PATENTSCHRIFT**

(45) Veröffentlichungstag der Patentschrift:
**24.06.87**

(21) Anmeldenummer: **83890157.7**

(22) Anmeldetag: **09.09.83**

(51) Int. Cl.⁴: **A 61 B 10/00**

(54) **Einrichtung zur Entnahme von Abstrichen aus Körperhöhlen.**

(30) Priorität: **10.09.82 AT 3403/82**

(43) Veröffentlichungstag der Anmeldung:
**18.04.84 Patentblatt 84/16**

(45) Bekanntmachung des Hinweises auf die Patenterteilung:
**24.06.87 Patentblatt 87/26**

(84) Benannte Vertragsstaaten:
**BE CH DE FR GB IT LI LU NL SE**

(56) Entgegenhaltungen:
**FR - A - 2 447 177**
**US - A - 2 137 710**
**US - A - 3 491 747**
**US - A - 4 054 127**

(73) Patentinhaber: **Huber, Johannes, Dr., Wollzeile 2,
A-1010 Wien (AT)**
Patentinhaber: **Knogler, Wolfgang, Dr., Görgengasse 27,
A-1190 Wien (AT)**

(72) Erfinder: **Huber, Johannes, Dr., Wollzeile 2, A-1010 Wien
(AT)**
Erfinder: **Knogler, Wolfgang, Dr., Görgengasse 27,
A-1190 Wien (AT)**

(74) Vertreter: **Haffner, Thomas M., Dr. et al,
Patentanwaltskanzlei Dipl.-Ing. Adolf Kretschmer Dr.
Thomas M. Haffner Schottengasse 3a, A-1014 Wien (AT)**

ACTORUM AG

## Beschreibung

Die Erfindung bezieht sich auf eine Einrichtung zur Entnahme von Abstrichen aus Körperhöhlen, mit einem Schutzrohr, in welchem axial verschieblich und drehbar ein Betätigungsglied geführt ist, welches wenigstens zwei unter elastischer Vorspannung in radialer Richtung ausklappbare Schaber trägt, welche Schaber durch axiale Verschiebung des Betätigungsgliedes relativ zum Schutzrohr ausklappbar sind (siehe z.B. US-A-3 491 747, US-A-2 137 710).

Zur Entnahme von Abstrichen, insbesondere aus dem Cervical- oder Intrauterinbereich, ist bereits eine Reihe von Einrichtungen bekannt geworden. Die eingangs genannte Einrichtung wurde beispielsweise für die Früherkennung von Unterleibskrebs durch die Portio und die Cervix in das Corpus eingeschoben, worauf die Schaber durch relative Verschiebung des Betätigungsgliedes zum Schutzrohr ausgeklappt wurden und durch Drehung cytologische Proben entnommen wurden.

Nachteilig bei dieser bekannten Ausbildung ist es, dass bei der Einführung durch die Portio bereits eine Gefahr der Beschädigung des Gewebes des Muttermundes besteht und dass weiters bei unsorgfältiger Handhabung ein zu tiefes Einschieben und damit eine Beschädigung des Fundus, d.h. der Rückwand des Corpus, bestand. Bei einem Einführen durch die Portio wurden mit den scharfen Kanten des Hüllrohres bereits Gewebszellen abgestreift und die in der Folge durch die ausgeklappten Schaber im Corpus abgestreiften Gewebszellen wurden nach Zurückziehen des Betätigungsgliedes gemeinsam mit diesen zuerst abgestreiften Zellen ausgebracht. Eine Unterscheidung, ob es sich auf Grund der Analyse der cytologischen Proben um ein Cervical- oder Corpuskarzinom handelt, lässt sich mit diesen bekannten Einrichtungen nicht durchführen.

Die Erfindung zielt nun darauf ab, eine Einrichtung der eingangs genannten Art dahingehend zu verbessern, dass eine Gefahr der Verletzung des Gewebes in unmittelbarer Nähe derjenigen Stelle, an welcher Proben entnommen werden sollen, vermieden wird, und zielt weiters darauf ab, bei der speziellen Verwendung zur Herstellung von Abstrichen im Cervical- bzw. Intrauterinbereich eine genauere Unterscheidung derjenigen Stellen zu ermöglichen, an welchen Gewebe tatsächlich abgenommen wurde. Zur Lösung dieser Aufgabe besteht die Erfindung im wesentlichen darin, dass das Schutzrohr an seinem den Schabern zugewendeten Ende an seinem Aussenumfang längs einer Schraubenlinie verlaufende Rippen aufweist. Dadurch, dass das Schutzrohr an seinem Einführende, welches den Schabern zugewendet ist, längs einer Schraubenlinie verlaufende Rippen aufweist, lässt sich die Einführung des Schutzrohres wesentlich schonender vornehmen als bei den bekannten Einrichtungen, welche durch die entsprechende Körperöffnung gestossen werden mussten. Gleichzeitig lässt sich die Eindringtiefe exakter dosieren und es wird dadurch die Gefahr von Läsionen in hinter der Öffnung, durch welche die Einrichtung hindurchgeführt werden muss, liegenden Bereichen vermieden. In einfacher Weise kann die gewünschte Eindringtiefe durch entsprechende Markierungen am Hüllrohr festgelegt werden und die gewünschte Eindringtiefe kann dann exakt und reproduzierbar erzielt werden.

Vorzugsweise ist die erfindungsgemässe Ausbildung hiebei so getroffen, dass wenigstens ein Schaber an seinem freien Ende einen hohlkugeligen löffelartigen Ansatz trägt, dessen konkave Seite bei zurückgezogenem Betätigungsglied die axiale Öffnung des Schutzrohres abdeckt, wobei zur weiteren Schonung der Bereiche, durch welche die Einrichtung hindurchgeführt werden muss, bevor Gewebe entnommen wird, vorzugsweise der Aussendurchmesser des hohlkugeligen Ansatzes grösser ist als der Innendurchmesser des Schutzrohres und kleiner oder vorzugsweise gleich dem Kerndurchmesser des durch die schraubenlinienförmig verlaufenden Rippen ausgebildeten Gewindes. Durch die hohlkugeligen löffelartigen Ansätze lässt sich Gewebe an der gewünschten Stelle hinter der Öffnung, durch welche die Einrichtung eingeführt wird, entnehmen und die hohlkugelige Gestalt sichert eine schonende Durchführung der Einrichtung durch die Körperöffnung. Zellen im Bereich der Durchführungsstelle des Körpers werden hiebei durch die Rippen des schraubenlinienförmigen Gewindes abgestreift und es lässt sich auf diese Weise bei Einführung der Einrichtung in den Uterusbereich eine Unterscheidung zwischen cervical entnommenen und intrauterin entnommenen Zellen vornehmen, da die intrauterin entnommenen Zellen nach Zurückziehen des Betätigungsgliedes geschützt im Inneren der hohlkugeligen Ansätze und des Schutzrohres ausgebracht werden, wohingegen die Zellen aus dem Bereich der Portio an den schraubenlinienförmig verlaufenden Rippen haften und mit dem Schutzrohr ausgebracht werden.

Zur Verbesserung der Aufnahme von Zellen kann die Ausbildung so getroffen sein, dass ein weiterer Schaber einen hohlkugeligen löffelartigen Ansatz trägt, dessen Aussendurchmesser höchstens gleich dem Innendurchmesser des hohlkugeligen löffelartigen Ansatzes des anderen Schabers ist. Eine deutlich verbesserte Schabwirkung ergibt sich dadurch, dass die Schaber nach ihrem Ausklappen in Ebenen liegen, welche den bei der Drehung des Betätigungsgliedes entstehenden Hüllkegel unter spitzen Winkeln schneiden.

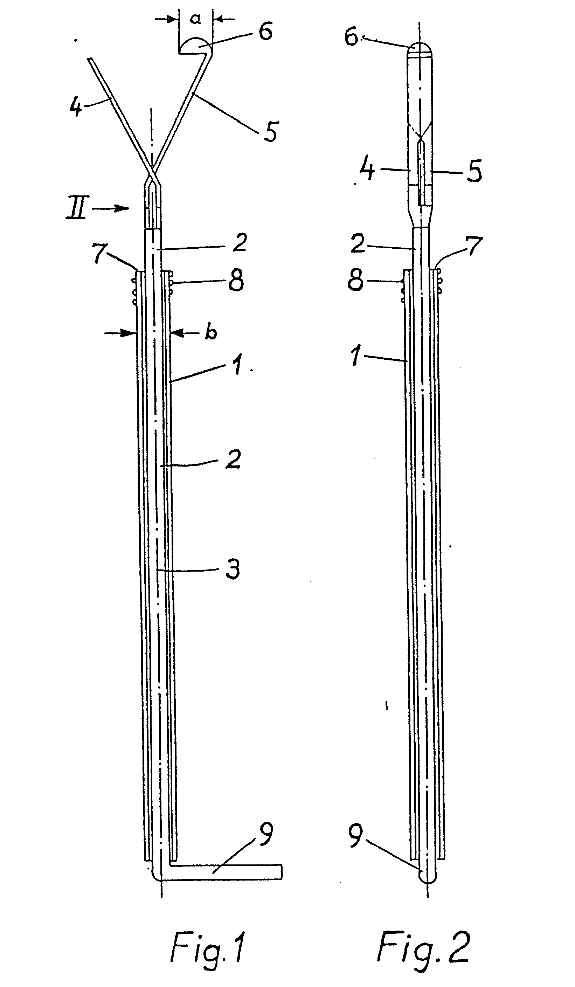

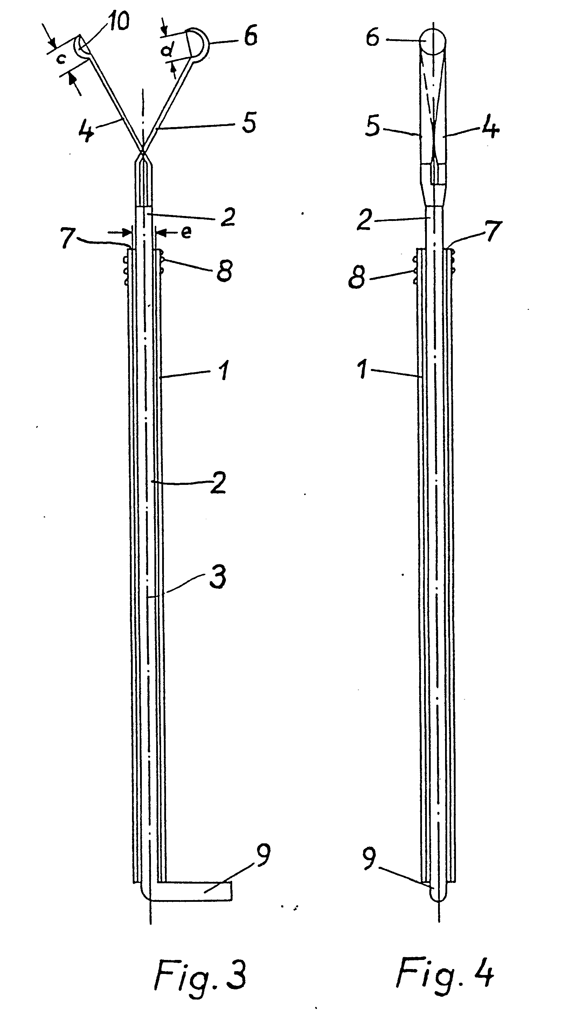

Die Erfindung wird nachfolgend anhand von in der Zeichnung dargestellten Ausführungsbeispielen näher erläutert. In dieser zeigen Fig. 1 eine Seitenansicht der erfindungsgemässen Vorrichtung teilweise im Schnitt, Fig. 2 die Vorrichtung nach Fig. 1 in der Ansicht entsprechend dem Pfeil II der Fig. 1, gleichfalls teilweise im Schnitt, Fig. 3 eine abgewandelte Ausführungsform in der gleichen Ansicht wie Fig. 1 und Fig. 4 eine Ansicht der Ausführungsform nach Fig. 3 in analoger Darstellung wie Fig. 2.

In Fig. 1 und 2 ist ein Schutzrohr mit 1 bezeichnet und es ist ein Betätigungsglied 2 vorgesehen, welches in Richtung seiner Achse 3 verschieblich und um die Achse 3 drehbar im Schutzrohr 1 gelagert ist. Das Betätigungsglied 2 trägt Schaber 4 und 5, welche unter elastischer Vorspannung von der Achse 3 radial nach aussen klappbar sind, sobald das Betätigungsglied 2 in die in Fig. 1 dargestellte Endlage verschoben ist. Der Schaber 5 trägt hiebei an seinem freien Ende einen hohlkugeligen Ansatz 6, dessen Durchmesser a gleich dem Aussendurchmesser b des Schutzrohres ist. Das den Schabern 4 und 5 zugewendete Ende 7 des Schutzrohres 1 trägt schraubenlinienförmig verlaufende Rippen 8, mittels welcher das Schutzrohr 1 in die Körperöffnung hineingedreht werden kann. Das den Schabern gegenüberliegende Ende des Betätigungsgliedes 2 weist einen abgewinkelten Griffteil 9 auf, mittels welchem die Verdrehung der Schaber und damit die Entnahme von Zellmaterial erleichtert wird.

Bei der Ausbildung nach Fig. 3 und 4 wurden die Bezugszeichen gemäss Fig. 1 und 2 beibehalten. Bei dieser Ausbildung sind beide Schaber 4 und 5 mit hohlkugeligen Ansätzen 6 und 10 ausgestattet, wobei der Aussendurchmesser c des hohlkugeligen Ansatzes 10 maximal gleich gross dem Innendurchmesser d des hohlkugeligen Ansatzes 6 ist. Bei Zurückziehen des Betätigungsgliedes 2 klappt somit der hohlkugelige Ansatz 10 in den hohlkugeligen Ansatz 6 ein und da der Aussendurchmesser a des hohlkugeligen Ansatzes 6 grösser ist als die lichte Weite e des Schutzrohres 1, wird auch hier eine Endstellung beim Zurückziehen der Schaber 4 und 5 in das Hüllrohr sichergestellt.

## Patentansprüche

1. Einrichtung zur Entnahme von Abstrichen aus Körperhöhlen, mit einem Schutzrohr (1), in welchem axial verschieblich und drehbar ein Betätigungsglied (2) geführt ist, welches wenigstens zwei unter elastischer Vorspannung in radialer Richtung ausklappbare Schaber (4, 5) trägt, welche Schaber durch axiale Verschiebung des Betätigungsgliedes relativ zum Schutzrohr ausklappbar sind, dadurch gekennzeichnet, dass das Schutzrohr (1) an seinem den Schabern (4, 5) zugewendeten Ende (7) an seinem Aussenumfang längs einer Schraubenlinie verlaufende Rippen (8) aufweist.

2. Einrichtung nach Anspruch 1, dadurch gekennzeichnet, dass wenigstens ein Schaber (5) an seinem freien Ende einen hohlkugeligen löffelartigen Ansatz (6) trägt, dessen konkave Seite bei zurückgezogenem Betätigungsglied (2) die axiale Öffnung des Schutzrohres (1) abdeckt.

3. Einrichtung nach Anspruch 2, dadurch gekennzeichnet, dass der Aussendurchmesser (a) des hohlkugeligen Ansatzes (6) grösser ist als der Innendurchmesser (e) des Schutzrohres (1) und kleiner oder vorzugsweise gleich dem Kerndurchmesser des durch die schraubenlinienförmig verlaufenden Rippen (8) ausgebildeten Gewindes ist.

4. Einrichtung nach Anspruch 2 oder 3, dadurch gekennzeichnet, dass ein weiterer Schaber (4) einen hohlkugeligen löffelartigen Ansatz (10) trägt, dessen Aussendurchmesser (e) höchstens gleich dem Innendurchmesser (d) des hohlkugeligen löffelartigen Ansatzes (6) des anderen Schabers (5) ist.

5. Einrichtung nach einem der Ansprüche 1 bis 4, dadurch gekennzeichnet, dass die Schaber (4, 5) nach ihrem Ausklappen in Ebenen liegen, welche den bei der Drehung des Betätigungsgliedes entstehenden Hüllkegel unter spitzen Winkeln schneiden.

## Claims

1. Curette device for taking swabs from body cavities, comprising a tubular body (1) in which an actuating stem (2) is guided reciprocably and rotatably carrying at least two scrapers (4, 5) capable to being swung out under resilient bias in radial direction by axial movement of the actuating stem relatively to the tubular body, characterised in that the end (7) of the tubular body (1) proximal to the scrapers (4, 5) at its periphery has helical ribs (8).

2. Curette device as claimed in claim 1, characterised in that at least one scraper (5) at its free end carries a hollow-spherical spoon-like attachment (6) the hollow side of which closes the axial mouth of the tubular body (1) when the actuating stem (2) is in its retracted position.

3. Curette device as claimed in claim 2, characterised in that the outer diameter (a) of the hollow-spherical attachment (6) is greater than the inner diameter (e) of the tubular body (1) and less than or preferably equal to the core diameter of the thread formed by the helical ribs (8).

4. Curette device as claimed in claim 2 or 3, characterised in that a further scraper (4) carries a hollow-spherical spoon-like attachment (10) whose outer diameter (e) at maximum is equal to the inner diameter (d) of the hollow-spherical spoon-like attachment (6) of the other scraper (5).

5. Curette device as claimed in any one of claims 1 to 4, characterised in that the scrapers (4, 5) after having been swung out lie in planes which intersect the envelope cone, generated by rotation of the actuating stem, under sharp angles.

## Revendications

1. Dispositif pour effectuer des prélèvements dans des cavités du corps, avec un tube de protection (1) dans lequel est guidé un organe de manœuvre (2) mobile dans le sens axial et tournant lequel porte au moins deux lames (4, 5) pouvant être pivotées, sous l'effet d'une précontrainte élastique, dans le sens radial, la sortie des lames étant obtenue par le déplacement axial de l'organe de manœuvre par rapport au tube de protection, caractérisé en ce que le tube de protection (1) comporte à son extrémité (7) dirigée vers les lames (4, 5), sur sa circonférence extérieure, des nervures (8) qui s'étendent le long d'une ligne hélicoïdale.

2. Dispositif selon la revendication 1, caractérisé en ce qu'au moins une lame (5) porte à son extrémité libre un prolongement sphérique creux (6) en forme de cuillère dont le côté concave recouvre l'ouverture axiale du tube de protection (1) lorsque l'organe de manœuvre (2) est rentré.

3. Dispositif selon la revendication 2, caractérisé en ce que le diamètre extérieur (a) du prolongement sphérique creux (6) est supérieur au diamètre intérieur (e) du tube de protection (1) et inférieur ou, de préférence, égal au diamètre du noyau du filetage formé par les nervures (8) hélicoïdales.

4. Dispositif selon l'une des revendications 2 ou 3, caractérisé en ceu qu'une autre lame (4) porte un prolongement sphérique creux (10) en forme de cuillère dont le diamètre extérieur (e) est au plus égal au diamètre intérieur (d) du prolongement sphérique creux (6) de l'autre lame (5).

5. Dispositif selon l'une quelconque des revendications 1 à 4, caractérisé en ce que, après leur déploiement, les lames (4, 5) se situent dans des plans qui coupent sous des angles aigus le cône enveloppant généré lors de la rotation de l'organe de manœuvre.

Fig.1          Fig.2

0 105 863

Fig. 3

Fig. 4

7